# EUROPEAN PATENT SPECIFICATION

(11) **EP 3 952 717 B1**
(45) Date of publication and mention of the grant of the patent: **04.11.2026**
(21) Application number: 20775431.8
(22) Date of filing: 07.04.2020
(51) Int. Cl.: A61B 1/00, A61B 1/005

(54) **STEERABLE INSTRUMENT COMPRISING A DETACHABLE PART**
LENKBARES INSTRUMENT MIT EINEM ABNEHMBAREN TEIL
INSTRUMENT ORIENTABLE COMPRENANT UNE PARTIE AMOVIBLE

(30) Priority: 08.04.2019 NL 2022893; 08.04.2019 NL 2022896; 11.10.2019 NL 2023998
(43) Date of publication of application: 16.02.2022
(73) Proprietor: Fortimedix Assets II B.V., 6167 RD Geleen (NL)
(72) Inventor: THISSEN, Mattheus Hendrik Louis, 6071 NC Swalmen (NL); VERBEEK, Marcel Antonius Elisabeth, 6367 HA Voerendaal (NL)
(74) Representative: Nederlandsch Octrooibureau
(86) International application number: PCT/NL2020/050239
(87) International publication number: WO 2020/218921

(56) References cited:
- WO-A1-2015/151093
- WO-A1-2017/068404
- WO-A1-2017/125036
- WO-A2-2020/218920
- JP-A- 2015 164 591
- US-A- 5 007 406
- US-A- 5 618 294
- US-A1- 2007 250 113
- US-A1- 2011 022 078
- US-A1- 2011 213 347
- US-A1- 2012 265 214
- US-A1- 2012 277 534
- US-A1- 2013 060 088
- US-A1- 2013 102 846
- US-A1- 2015 057 610
- US-A1- 2015 366 445
- US-A1- 2016 278 616
- US-A1- 2017 273 702
- US-A1- 2018 071 490

## Description

### Field of the invention

The present invention relates to a steerable instrument for invasive and non-invasive type of applications, such as in surgery. Such instruments can be used in, for instance, the field of gastroscopy, colonoscopy, endoscopy, laparoscopy, and other medical applications. However, the steerable instrument according to the invention can also be used in non-medical applications. Examples of the latter include inspection and/or repair of mechanical and/or electronic hardware at locations that are difficult to reach.

### Background art

Transformation of surgical interventions that require large incisions for exposing a target area into minimal invasive surgical interventions, i.e. requiring only natural orifices or small incisions for establishing access to the target area, is a well-known and ongoing process. In performing minimal invasive surgical interventions, an operator such as a physician, requires an access device that is arranged for introducing and guiding invasive instruments into the human or animal body via an access port of that body. In order to reduce scar tissue formation and pain to a human or animal patient, the access port is preferably provided by a single small incision in the skin and underlying tissue. In that respect the possibility to use a natural orifice of the body would even be better. The access device preferably enables the operator to control one or more degrees of freedom that the invasive instruments offer. In this way, the operator can perform required actions at the target area in the human or animal body in an ergonomic and accurate manner.

Steerable surgical invasive instruments in the field of gastroscopy, colonoscopy, endoscopy, laparoscopy, etc. are well-known in the art. The invasive instruments can comprise a steerable tube shaped device that enhances its navigation and steering capabilities. Such a steerable tube shaped device may comprise a proximal end part, a distal end part including at least one deflectable zone, and a rigid or flexible intermediate part or shaft, wherein the steerable tube shaped device, at its proximal end, further comprises a steering arrangement that is adapted to deflect the distal deflectable zone relative to a central axis of the tube shaped device.

Most of the known instruments are complex to manufacture resulting in expensive instruments. Often, the distal end of the instruments comprise a flexible zone that is composed of separate links with hinging pins, coils or flexible plastic extrusions. Steering cables should be guided through holes through these links and/or through guiding eyes or hooks.

In many prior art devices, the steering arrangement comprises conventional steering cables with, for instance, sub 1 mm diameters as control members, wherein the steering cables are arranged between related deflectable zones at the distal end part and the steering arrangements at the proximal end part of the tube shaped device. Alternatively, control members may be implemented by one or more sets of longitudinal elements that are, e.g., formed by laser cutting in tube elements. Further details regarding the design and fabrication of the abovementioned steerable tube and the steering arrangement thereof have been described for example in WO 2009/112060 A1, WO 2009/127236 A1, WO 2017/213491 A1, and WO 2018/067004. Such instruments can advantageously be used in endoscopic operations where the length need not be more than say 1 meter.

US2015366445A1 discloses a steerable endotracheal intubation/endoscopic device having a shaft with a flexible steerable distal end controlled by proximal control cables and a steering control mechanism. The device further includes a locking/unlocking mechanism for the control cables, allowing cable tension to be reset when the shaft is bent into a new configuration.

Sometimes a plastic extruded tube can be used with integrated channels for accommodating the cables. This renders an instrument with a simple construction. However, most plastics are rather weak. In case of very long instruments, e.g. longer than 1 meter, therefore, problems may arise due to the high forces exerted on the cables, both the steering cables and the actuation cable arranged to operate the tool at the distal end of the instrument. Problems may be undesired cuts, slip stick effects in the plastic tube and often a too high friction on the cables causing steering by the steering cables to be difficult and hard to manage. Moreover, mechanical properties of many plastics may be too poor to guarantee a high enough torsion stiffness which is required because the instruments should be capable of being rotated in use where they may have been guided through several curves impeding rotation of the whole instrument. Another disadvantage of a plastic tube may be that in case it is provided with an actuation cable to operate a tool at the distal end of the instrument the force in the actuation cable can increase to an extent that it exceeds the maximum longitudinal force allowed in the extruded plastic tube. If so, it would be impossible to operate the tool with an acceptable force. Moreover, if the plastic tube is in a curved arrangement and high force is exerted on the actuation cable, the channels for the steering cables may be deformed, especially in bent/deflected portions, such that the steering cables are clamped and cannot move freely anymore in the channels, thus, impeding proper operation of the steering of the distal deflectable zones.

In medical applications where longer instruments are necessary, such as in colonoscopy where 1.5 meter long instruments (or longer) may be applied, requirements as to steerability, flexibility, stiffness and accuracy increase seriously. There is a desire to develop such instruments with a better performance than prior art devices as to steerability also under end-effector actuation, longitudinal stiffness, torsion stiffness, durability and applicability of a mechanically actuated tool at the distal end.

In medical applications, contamination of an instrument after it has been used to perform a surgical procedure on a patient can be a problem resulting in undesired post-operative complications. The contamination may be due to blood, other body fluids, tissue, etc. As a consequence of the contamination, the instrument may contain germs, viruses or other biological or chemical substances that could threat the health of the next patient on which the instrument is used.

One way of avoiding this contamination requires performing a thorough cleaning and sterilization of the instrument before each use. In many cases, the cleaning process is not capable of removing all contamination, and/or is very expensive. Therefore, a risk of adverse effects on a patient that is treated with such an instrument still exists. In order to prevent the risk of contamination, there is a preference for using disposable instruments which are used a single time and are thrown away after treating one patient.

### Summary

The invention is defined by independent claims 1 and 15 and their respective dependent claims. According to a first aspect of the disclosure, a steering device for connecting to an elongated instrument comprises a supporting unit comprising a first channel having a first central axis, said first channel extending from a distal side to a proximal side of the supporting unit, wherein the supporting unit is arranged around said first channel, and a steering unit rotationally arranged on said supporting unit, the steering unit arranged to secure/relase a plurality of longitudinal elements of an instrument to move with the rotational movement of the steering unit.

Such a steering device can allow for easy coupling and decoupling of an instrument for medical or other operations. The elongated instrument can use the longitudinal elements for steering purposes, allowing for bending and movement at a distal end controlled by the steering unit. By having a steering unit to which the longitudinal elements secure, the longitudinal elements can be push or pull for proper steering, and this allows for easy connection and disconnection of the instrument from the steering device. This allows for the instrument to be quickly, securely and easily coupled to a steering device when needed, by even non-technical persons. With this simple connection, the instrument could be disposable while more complicated steering device and steering unit parts are reusable.

According to an embodiment, the supporting unit comprises a ball shaped element such that the steering unit can rotate around the ball shaped element and the first channel extends through the ball shaped element. Such a ball-shaped element can allow for the movement steering unit needs with respect to the instrument shaft while preventing any movements which could cause twisting or tangling of the longitudinal steering elements.

According to an embodiment, the steering unit comprises a steering plate and one or more frames connected around one or more axes to enable movement of the steering plate in space. By enabling any movement of steering plate, the movement of the instrument can be more precisely controlled.

According to an embodiment, the steering unit comprises a second channel and the ball shaped element is arranged in said second channel, thereby allowing the steering device to control the movement of instrument very precisely.

According to an embodiment, the steering unit comprises a plurality of connectors for connecting the plurality of longitudinal elements to the steering unit, thereby enabling pushing and pulling movements by the steering device for controlling the movement of instrument very precisely. The plurality of connectors may comprise a plurality of openings in the steering unit configured to receive one longitudinal element wherein the plurality of openings may be any suitable number and have any suitable shape. The plurality of connectors may comprise a plurality of grooves extending inward from the circumference of the steering unit. This allows for easy attaching and detaching of the longitudinal elements to the steering device.

According to an embodiment, the plurality of connectors may comprise a plurality of arm-shaped elements extending outwardly from the steering member unit. The arm-shaped elements can be easily connected to and disconnected from the instrument by, for instance, providing an end part or any other suitable part of the arm-shaped element that can be introduced in and extracted from the longitudinal elements of the instrument.

According to an embodiment, the steering device further comprises a locking mechanism for securing the longitudinal elements to the steering unit. This allows for avoiding that the longitudinal elements can be released accidentally during use by locking the locking mechanism, and easily release the instrument from the steering unit by opening the locking mechanism.

According to an embodiment, the locking mechanism comprises a locking plate configured to secure the plurality of longitudinal elements to the steering unit, wherein the plurality of longitudinal elements are secured to the steering unit through a plurality of openings and the locking plate connects to the steering unit such that the locking plate prevents the plurality of longitudinal elements from exiting the plurality of openings. Furthermore, the locking plate may be movable from an open position where the longitudinal elements can be inserted or removed from the openings to a locked position where the longitudinal elements are secured within the openings. These are advantageous ways of allowing to attach and detach the instrument and the steering device.

The locking plate may be moveable axially to secure longitudinal elements in openings extending inward from the circumference of the steering unit. Furthermore, the locking plate is rotationally moveable to move from the open position to the locked position.

In another embodiment, the locking plate comprises a plurality of protruding elements extending around the outer circumference of the locking plate, wherein each of the plurality of protruding elements comprises a hook-shaped side arranged to receive a longitudinal element in a channel to secure the longitudinal element. In this way, the longitudinal elements can be easily secured by rotating the locking device in the right direction. Instead of a hook-shaped side, the protruding elements may have any other shape that allows the longitudinal elements to be secured in the channel. The protruding elements may comprise an inclined side arranged opposite to the channel such that when the locking plate rotates from the locked position with the longitudinal elements secured in the channels to the open position, the longitudinal elements slide on the inclined side of the protruding elements and are thereby pushed out from the plurality of openings. This provides a very simple and advantageous way of releasing the instrument form the steering device. The steering device may be arranged such that, when the plurality of elongated elements are secured to the steering unit, rotational movement of the steering unit around the supporting unit will cause the steering unit to push and/or pull the plurality of elongated elements

According to an embodiment of the disclosure, an elongated instrument for connecting to a steering device comprises an elongated shaft with a distal end and a proximal end, and a plurality of elongated elements extending along the shaft, wherein each of the plurality of elongated elements comprises at the proximal end a connecting part for securing the elongated instrument to the steering device.

According to an embodiment of the disclosure, the plurality of longitudinal elements are arranged extending outwardly from the elongated shaft. This provides an advantage for easily connecting the instrument from a steering device because the longitudinal elements are already arranged at the correct attaching position. Furthermore, the longitudinal instrument may comprise a cone-shaped device at the proximal end arranged to push away the longitudinal elements for extending outwardly from the elongated shaft. This allows the longitudinal elements to stay in the correct attaching position.

According to a disclosed example not covered by the claims, the connecting parts of the elongated instrument may comprise an opening. This allows for easy connection and disconnection of the instrument and the steering device because arm-shaped elements or any other suitable shaped element of the steering device can be inserting into or extracting from the openings of the longitudinal elements.

The elongated instrument comprises a tube and wherein the plurality of longitudinal elements are made by either laser or water cutting a plurality of longitudinal slots in the tube.

Embodiments of the invention are claimed in dependent claims.

Further features and advantages of the invention will become apparent from the description of the invention by way of non-limiting and non-exclusive embodiments. These embodiments are not to be construed as limiting the scope of protection. The person skilled in the art will realize that other alternatives and equivalent embodiments of the invention can be conceived and reduced to practice without departing from the scope of the present invention. Moreover, separate features of different embodiments can be combined, even if not explicitly shown in the drawings or explained in the specification, unless such combination is physically impossible. The scope of the present invention is only limited by the claims.

### Brief description of the drawings

The present invention will be discussed in more detail below, with reference to the attached drawings, in which like or same reference symbols denote like, same or corresponding parts, and in which:
Fig. 1 shows an instrument according to one embodiment of the invention.
Fig. 2 shows the instrument of Fig. 1 with the longitudinal elements arranged extending outwardly.
Fig. 3 and 4 show alternative structures for the embodiment of Fig. 2.
Fig. 5 shows an instrument and a steering device according to one embodiment of the invention
Fig. 6 shows the instrument and the steering device of Fig. 5 coupled to each other.
Fig. 7 shows an alternative structure for the steering device of Fig. 5.
Fig. 8A and 8B show the instrument and the steering device of Fig. 6 further comprising a locking mechanism.
Fig. 9A, 9B and 9C show an alternative structure of the locking mechanism of Fig. 8 in three different positions.
Figs. 10A-10B shows an embodiment of a steering device with an instrument being coupled to the steering device.
Figs. 10C-10D show cross-sectional views of the coupling shown in Figs. 10A-10B.
Figs. 11A-11C another embodiment of a steering device with an instrument being coupled to the steering device.
Fig. 12 shows the full instrument with steering device, connected and with a deflection of the distal end.

### Description of embodiments

Same reference number will be used in different figures for indicating the same element.

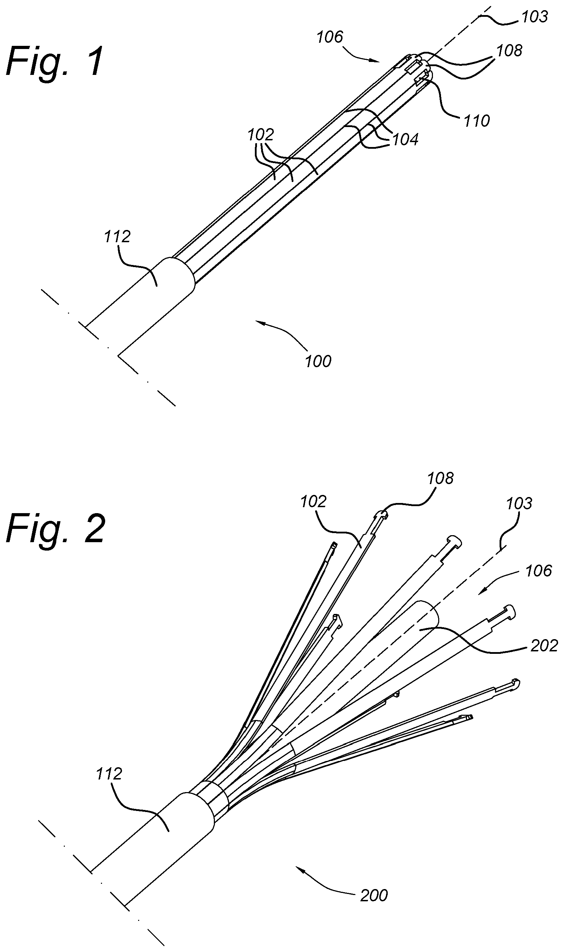

Figure 1 shows a non limiting embodiment of an instrument 100 comprising a plurality of longitudinal elements 102 arranged around a central axis 103 of the instrument 100. Instrument 100 is shown schematically to represent any steerable instrument for use in medical or other operations. Suitable instrument arrangements can be found in NL2021823, titled Steerable instrument comprising a tube element, Filed on Oct. 16, 2018 and hereby incorporated by reference. The longitudinal elements 102 shown in Figure 1 are implemented by longitudinal strip shaped elements in tube elements and separated by longitudinal slots 104 resulting from laser cutting in the cylindrical tube, as explained in detail in, for example, WO 2009/112060 A1, WO 2009/127236 A1, WO 2017/213491 A1, and WO 2018/067004, or by rigid cables or wires.

These longitudinal slots 104 extend through the whole thickness of the material. As an alternative to laser cutting other techniques may be used, e.g., cutting by means of water jets. Also, other methods such as 3D laser printing may be used. These slots extend through the whole thickness of the material. This also holds for other embodiments discussed below where reference is made to laser cutting.

The longitudinal elements 102 comprise at a proximal end 106 of the instrument 100 a T-shaped connecting part 108 which may be implemented by laser cutting a rectangular slot 110 at the proximal end 106 of each longitudinal slot 104. The instrument 100 shown in Figure 1 may comprise further at least a distal deflectable zone at a distal end of the instrument 100 (not shown in Figure 1) wherein the longitudinal elements 102 are connected to the distal deflectable zone (not shown in Figure 1) such that by pulling or pushing the longitudinal elements 102 the distal deflectable zone is controlled to deflect in a desired direction. A tool (not shown in Figure 1), like a forceps or any other kind of suitable tool, may be arranged at said distal end of the instrument 100. The instrument 100 shown in Figure 1 may also comprise an outer tube 112 covering partially or completely the longitudinal elements 102. Furthermore, the instrument 100 may comprise as well a shaft (not shown in Figure 1) arranged inside the shaft 112 such that the longitudinal elements 102 are arranged surrounding said shaft 112.

Figure 2 shows a non limiting embodiment of the instrument shown in Figure 1 wherein the longitudinal elements 102 are arranged to extend outwardly from the shaft 202 at the proximal end 106 of the instrument 200. The longitudinal elements 102 may be arranged to extend outwardly by manually bending or by any other suitable means.

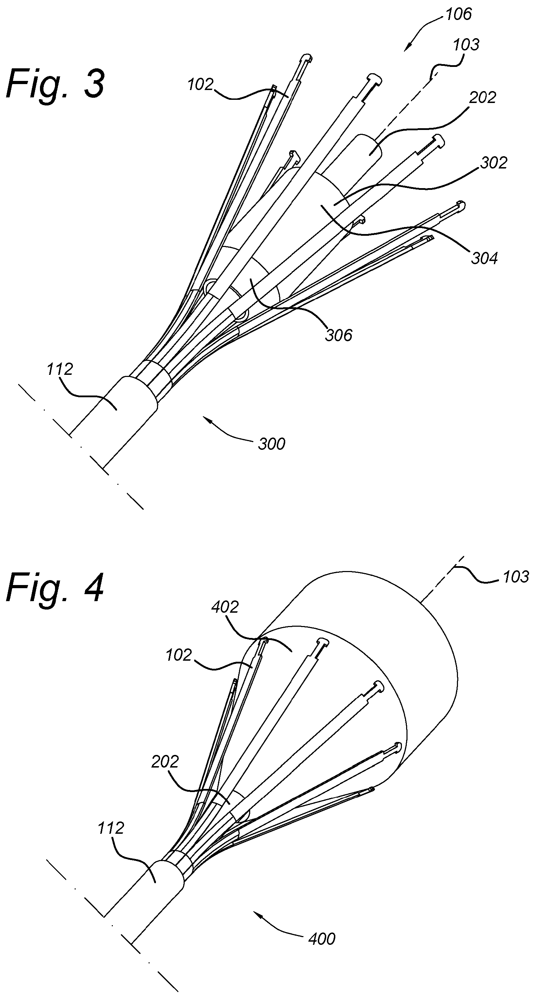

Figure 3 shows an alternative non limiting embodiment of the instrument shown in Figure 2 wherein the instrument 300 comprises a device 302 arranged around the shaft 202 wherein the device 302 comprises a proximal end 304 and a distal end 306 wherein the proximal end 304 comprises a cylindrical shape and the distal end 306 comprises a conical shape such that the conical shape is arranged to push the longitudinal elements 102 outwardly from the shaft 202.

Figure 4 shows an alternative example method of expanding the longitudinal elements of the instrument shown in Figure 2. Longitudinal elements 102 are arranged extending outwardly from the central axis 103 of the instrument 400 by a cone-shaped device 402 wherein the cone-shaped device 402 is arranged surrounding the shaft 202 such that the longitudinal elements 102 are expanded by the outer surface of the cone-shaped device 402.

It should be understood that any other suitable device or method can be used to arrange the longitudinal elements at the proximal end such that the longitudinal elements extend outwardly from the central axis of the instrument.

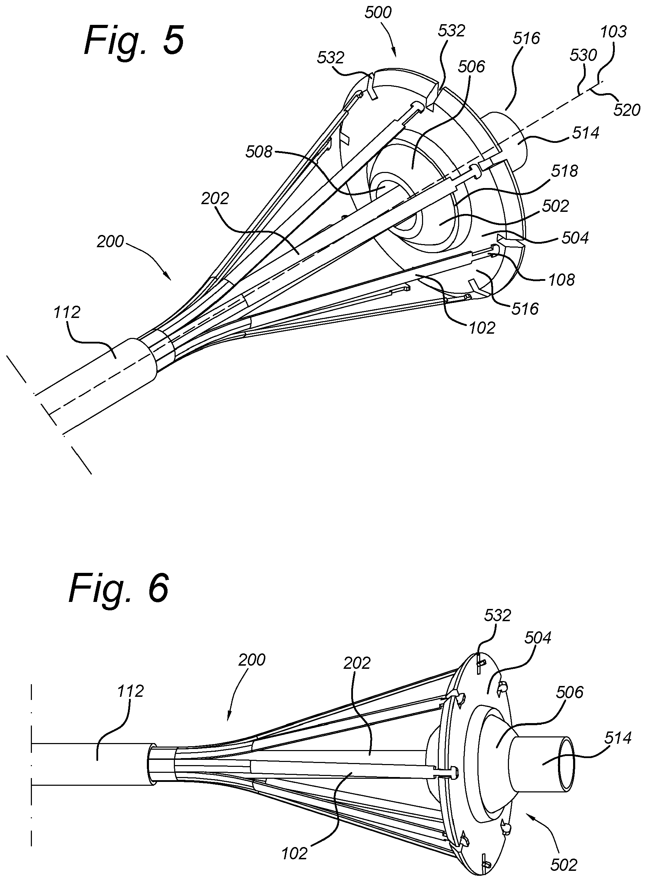

Figure 5 shows a steering device 500 according to one embodiment with an instrument being connected.

The steering device 500 comprises a supporting unit 502 and a steering unit 504. The supporting unit 502 comprises a ball-shaped element 506 traversed by a first channel 508 wherein the first channel 508 has a circular shape and extends from a proximal end 510 of the steering device 500 to a distal end 512 of the steering device 500. The first channel 508 extends from the ball-shaped element 506 at the proximal end of the steering device 500 thereby defining a hollow tube 514 which extends outwardly from the ball-shaped element.

The steering unit 504 comprises a steering plate 516 and a second channel 518 located at the centre of the steering plate 516 wherein the second channel 518 comprises a circular shape.

The supporting unit 502 is arranged in the second channel 518 such that the steering unit 504 can rotate around the ball-shaped element 506 and such that the central axis 520 of the first channel 508 coincides with the central axis 530 of the second channel.

The steering plate 516 comprises further a plurality of grooves 532 extending inward from the circumference of the steering unit of the steering plate 516 wherein the grooves 532 have a U-shape, though could take a number of different shapes in different embodiments.

The steering device 500 is arranged to be attached to the instrument 200 in the following way. Each elongated element include a connecting part 509 which can fit into groove 532 and secure in groove 532. In this case, a T-shaped connection is formed with a narrower portion to go within the groove and a wider portion at the end to ensure no longitudinal movement of elongated elements is allowed once connected. The inner shaft 406 of the instrument is introduced into the side of the first channel 504 of the supporting member 502 located at the distal end of the steering device 500 such that the central axis 409 of the instrument coincides with the central axis 520 of the first channel 508 and such that the connecting part 509 of the longitudinal elements 408 align with the grooves 532. The connecting part 509 of the longitudinal elements 408 is then pushed into the grooves such that the upper wider part of the T shape is secured at a proximal side of the steering plate, as shown in Fig. 6.

As it can be seen in Fig. 6, by moving the steering plate 516 around the ball-shaped element 506, the longitudinal elements 408 are pushed or pulled by the steering plate 516 thereby causing distal deflection (not shown in Fig. 6).

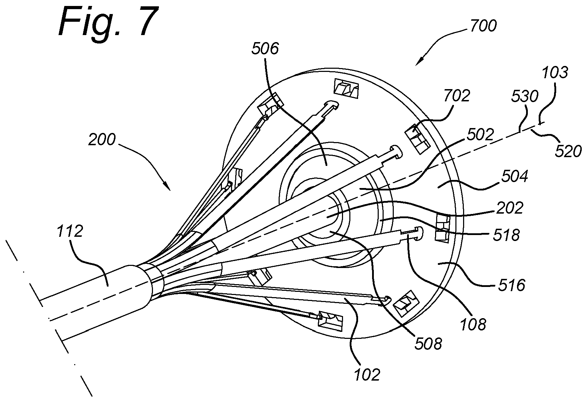

Figure 7 shows an alternative embodiment of the steering device 500 of Fig. 5 wherein the steering plate 516 comprises a plurality of openings 702 instead of circumferential grooves. The way of attaching the instrument to the steering device 700 is similar to that explained with reference to Fig. 5 but in this embodiment the connecting parts 108 of the longitudinal elements are pushed against the openings, such that they "click" into a receiving portion of the steering plate 516. As can be seen in this embodiment, the connecting portions click into a complementary shaped receiving portion in the steering plate 516, with a central opening and a ramp to guide the catching feature (here, the wider "top" of the connecting portion 509) to a securing position.

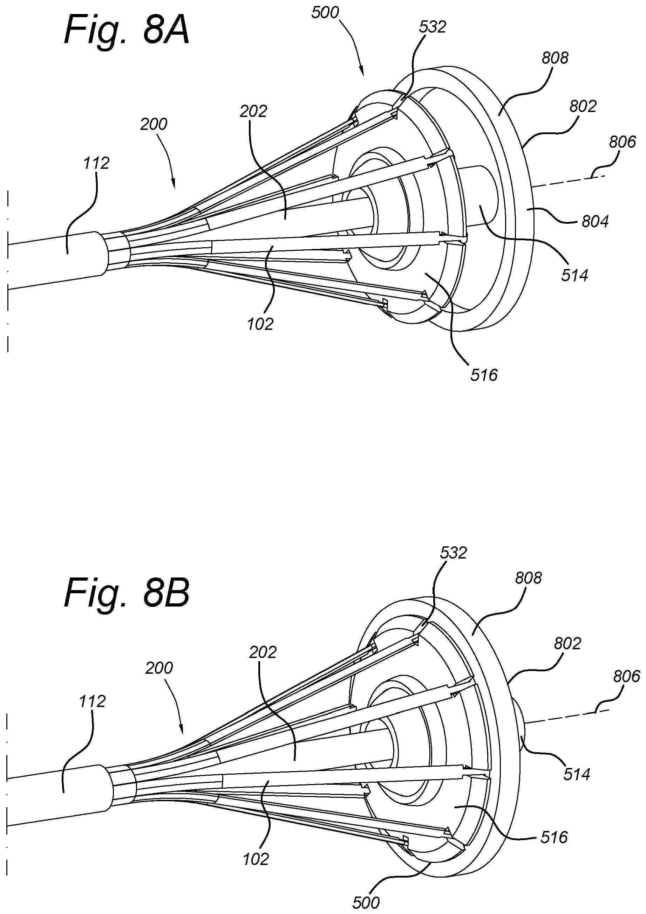

Figure 8A and 8B shows the embodiment of Fig. 6 further comprising a locking mechanism in the form of locking plate 804.

The locking mechanism 802 shown in Figs. 8A and 8B is movable between an open position (shown in Fig. 8A) and a closed position (shown in Fig. 8B).

As it can be seen in Fig. 8A, the steering device 500 and the instrument 200 are coupled to each other such that the elongated elements 102 are placed in the grooves 532 as it has been explained with reference to Fig. 5. Fig. 8A also shows a locking mechanism 802 for securing the longitudinal elements 102 to the steering unit 504 wherein the locking mechanism 802 is in an open position such that the longitudinal elements 102 can be inserted into or removed from the grooves 532. The locking mechanism 802 comprises a locking plate 804 having a circular shape with an opening in the centre such that the tube 514 of the supporting unit 502 can pass through the opening. The locking plate 804 is movable axially from an open position where the longitudinal elements are removed from the grooves to a locked position wherein the longitudinal elements are secured within the grooves, e.g, the locking plate 804 can be moved towards or away the steering device 500 along the central axis 806 and/or could be moved in a rotational manner.

Fig. 8B shows the embodiment of Fig. 8A wherein the locking mechanism has been moved from the open position shown in Fig. 8A to the locked position of Fig. 8B by moving the locking plate axially towards the steering device. This movement is until the locking plate 804 clicks against the steering plate 516 and/or connecting parts 509 of the longitudinal elements thereby preventing the longitudinal elements from being removed from the grooves 532 or openings 702. Locking mechanism and/or plate 804 can be in a number of different shapes as long as it can quickly and securely couple to steering plate 516 to ensure that longitudinal elements are secured to steering plate 516.

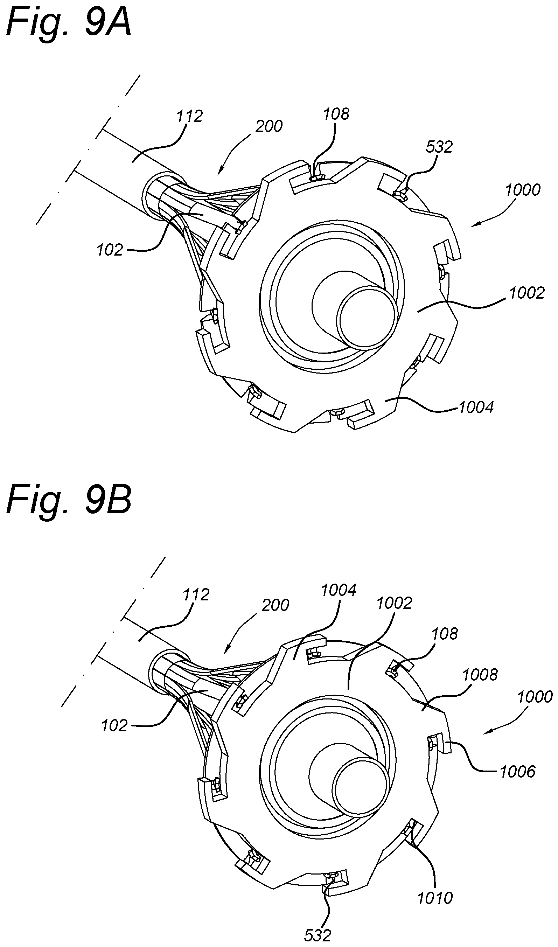

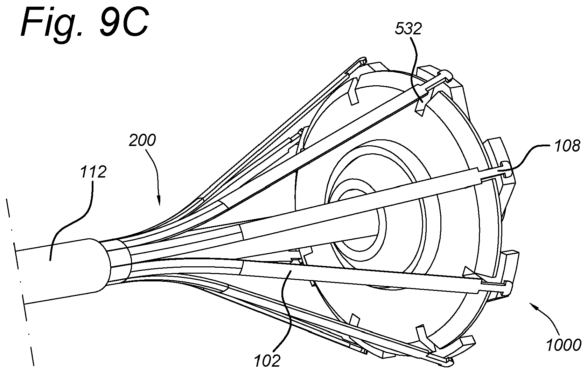

Fig. 9A, 9B and 9C show an alternative structure for the locking mechanism of Fig. 8.

The locking mechanism 1000 shown in Figs. 9A, 9B and 9C comprise a locking plate 1002. The locking plate 1002 comprises an opening arranged such that the locking plate 1002 can be rotationally mounted with respect to steering plate 516. The locking plate 1002 comprises a plurality of protruding elements 1004 extending around an outer circumference of the locking plate. Each of the protruding elements 1004 comprises a hook-shaped side 1006 which forms a channel and an inclined side 1008.

Fig. 9A shows the locking mechanism 1000 in a position wherein each longitudinal element 102 is located between the hooked-shape side 1006 of a protruding element and the inclined side of the neighboring protruding element such that the longitudinal elements 102 can be removed from the grooves 532 of steering plate 516.

The locking plate 1002 is configured to be axially clockwise rotated from the open position shown in Fig. 9A to the locked position shown in Fig. 9B. The locking plate 1002 shown in Fig. 9A is axially clockwise rotated such that the hooked-shape side of each of the protruding elements moves towards the corresponding elongated element 102 until the elongated element is inside of a channel 1010 defined by the hooked-shape side thereby reaching the locked position shown in Fig. 9B wherein the longitudinal elements 102 are secured in the channels 1010.

Fig. 9C shows the locking device shown in Fig. 9B after being axially clockwise rotated from the locked position to a detached position wherein the longitudinal elements have been removed from the grooves. By axially rotting the locking plate counter-clockwise, the inclined side of each of the protruding elements moves towards the corresponding longitudinal element 102 such that the longitudinal element slides on the inclined side and is pushed out from its corresponding groove by the inclined side 1008, thereby reaching the detached position shown in Fig. 9C wherein the longitudinal elements 102 are out of the grooves.

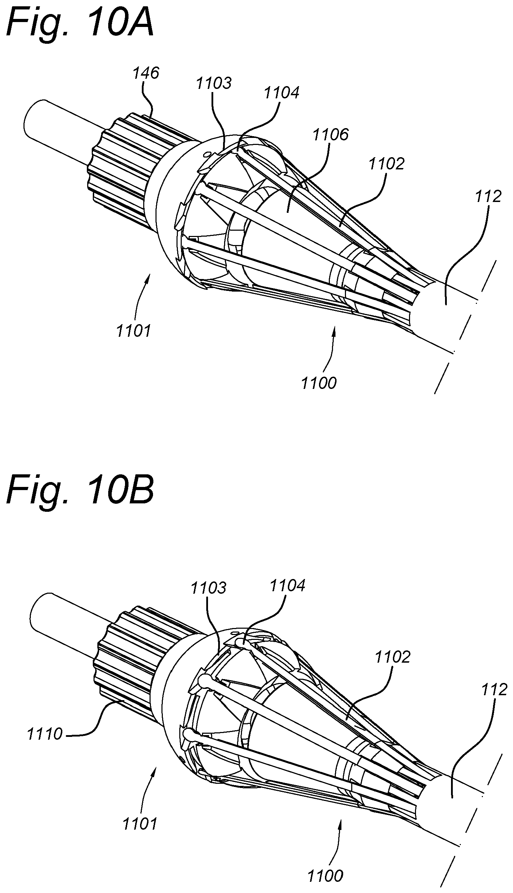

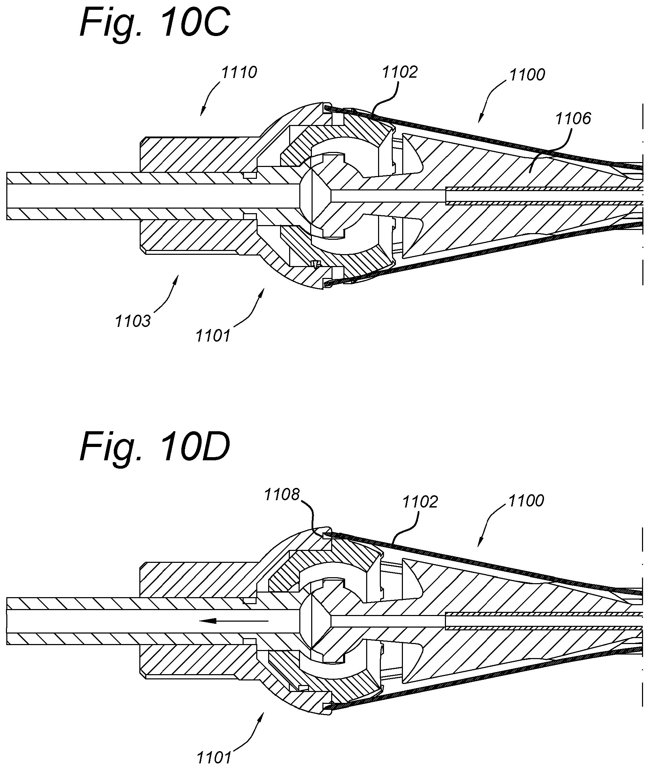

Figs. 10A-10D shown another embodiment according to the invention comprising an instrument 1100, a steering device 1101 and a locking mechanism 1103. Figs. 10A and 10B show the instrument and the steering device coupled together wherein the locking mechanism is in an open position in Fig. 10A and in a closed position in Fig. 10B. Figs. 10C and 10D shown cross-sectional views of the instrument 1100 and steering device being coupled together.

Figs. 10A-10D show an instrument 1100 like the instrument in Fig. 1 wherein the connecting parts 1104 of the longitudinal elements 1102 have a circular shape instead of a T-shape.

The locking mechanism 1103 shown in Figs. 10A-10D comprises a semi spherical shape with a locking portion which operates similar to locking plate 1002 shown in Figs. 9A-9C. Locking mechanism 1103 includes slots for receiving connecting portions 1104 or longitudinal elements 1102. Longitudinal elements 1102 can be slide into place by following the curvature of conical section 1106 leading to receiving grooves 1108. As can be seen in Fig. 10C, conical section 1106 is moved closer to locking mechanism 1103 when in an open position. This allows for easier insertion (and removal of elongated elements) into grooves 1108. When elongated elements 1102 are secured in grooves, locking mechanism 1103 is rotated to secure elongated elements in place in grooves. This is shown in Fig. 10A and 10D. Such rotational movement to a locked position also extends conical section 1106 axially with respect to locking mechanism 1103. This ensures proper tensioning of elongated elements, to ensure proper bending and deflection of the instrument. The instrument can be easily removed in the same steps, rotation of locking mechanism 1103, followed by pulling instrument axially away from steering device 1101. When locking mechanism 1103 is rotated to the open position, as seen in Fig. 10B, elongated elements 1102 are pushed from grooves 1108 for easy detachment of instrument from steering device. Rotation could be performed manually, for example, through use of gripping portion, or could be automated.

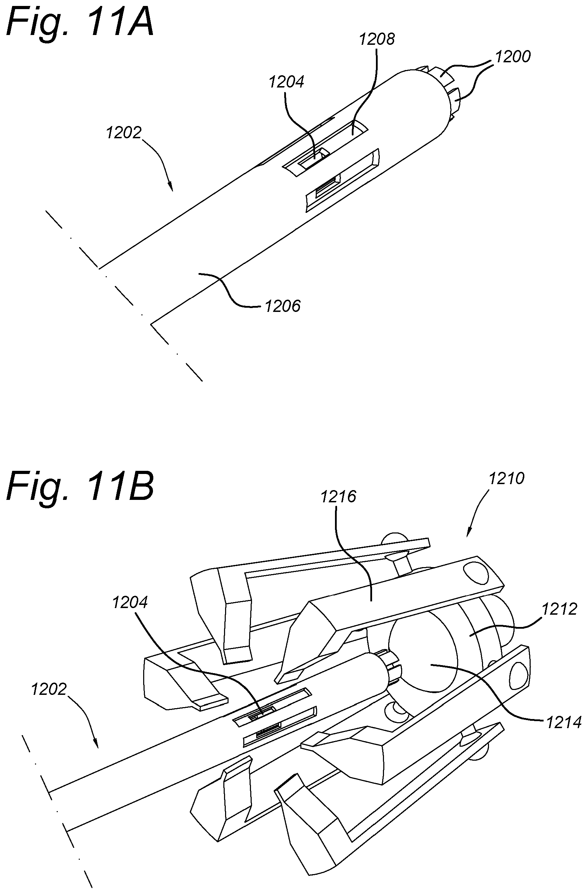

Figs. 11A-11C shows an alternative structure for an instrument and a steering device according to another example not covered by the claims.

The instrument 1202 comprises an outer tube 1206 covering the longitudinal elements 1200. The outer tube 1206 comprises a plurality of openings 1208. The longitudinal elements 1200 of instrument 1202 also comprise a plurality of openings 1204 overlapping with the openings 1208 of the outer tube 1206. The openings 1208 of the outer tube and the openings 1204 of the longitudinal elements 1200 may result from laser cutting in the corresponding cylindrical tubes.

These openings 1204 and 1208 extend through the whole thickness of the material. As an alternative to laser cutting other techniques may be used, e.g., cutting by means of water jets. Also, other methods such as 3D laser printing may be used.

Figs. 11B shows the instrument 1202 and a steering device 1210 wherein the steering device 1210 and the instrument 1202 are detached. The steering device 1210 comprises a steering unit 1212 and a supporting unit 1214 wherein the steering unit 1212 is rotationally mounted on the supporting unit 1214 similar to the supporting unit 502 and the steering unit 504 of Fig. 5. The steering unit 1212 comprises a plurality of a plurality of arm-shaped elements 1216 fixedly connected to the steering unit 1212 and extending outwardly from the steering unit for connecting the plurality of longitudinal elements 1208 to the steering unit 1212.

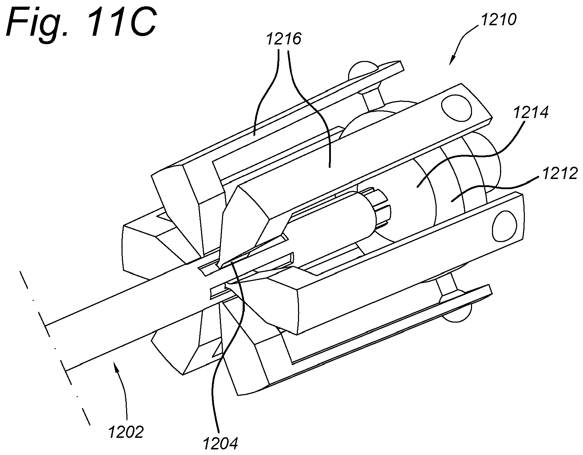

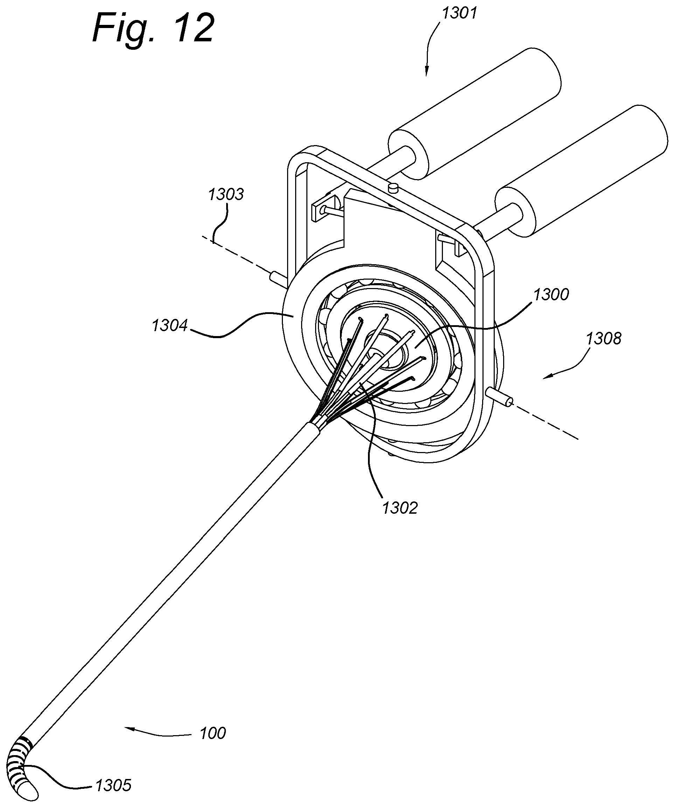

Fig. 11C shows the instrument 1202 and the steering device 1210 of Fig. 11B connected together by inserting the end part of the arm-shaped elements 1216 into the openings 1204 of the longitudinal elements 1200 such that, by steering the steering unit 1212 around the supporting unit 1214, the arm-shaped elements 1216 pull from or push the longitudinal elements 1200 for controlling the distal end (not shown in Figs. 12A-C) of the instrument 1202.

Fig. 12 shows an example of a deflection of steering plate 1300, causing a deflection in a distal end 1305 of instrument 100. Actuators 1301 are connected to inner frame 1304 around a hinge axis 1303. Other embodiments could include another frame with another hinge axis. To enable any movement of the steering plate 1300 in space. The steering unit could be controlled manually, or through robotics, or a combination of the two. Actuators 1301 move inner frame 1304 such that the distal end is deflected in a certain direction. This causes tension in some longitudinal elements and relaxation in others, thereby causing the deflection at the distal end. Some embodiments could include the longitudinal elements connected differently at the distal end (e.g., rotated 180 degrees) such that they cause opposite deflection of the distal end for the same movement depicted in steering unit 1308.

As can be seen in Fig. 12, in order to enable movement in any three dimensional plane as well as rotational movement of steering plate 1300, and therefore any bending or rotation of instrument 100, the suspension and control through steering device 1308 can be complicated. By using a steering plate 1300 to which longitudinal elements 1302 of instrument secure, and providing a quick but simple and secure coupling to a locking plate, steering device 1308 can be reused while allowing for a disposable instrument. This enables more complicated steering devices 1308, particularly useful in robotics applications.

The different steerable instruments described in the different embodiments can include a handle arranged at the proximal end if manually operated (see Fig. 12), or can include a robotic steering unit for steering the distal end of the instrument and/or for manipulating a tool, arranged at the distal end of the instrument. Such a tool can, for example, be a camera, a manual manipulator, e.g. a pair of scissors, manipulators using an energy source, e.g. an electrical, ultrasonic or optical energy source. The instrument has no limitation as to the type of tool applied at the distal end. The type of handle or robotic steering unit and/or connection will be selected depending on the type of tool applied at the distal end, the intended use and other instrument or operational requirements.

Also the steering units have been described as ball-shaped, though could be shaped differently in different embodiments. In some embodiments steering plate of the steering unit may be connected differently (e.g., directly to channel or locking plate) and/or not include a support member. Longitudinal elements may be connected to different portions of the steering unit and in a variety of different ways, typically circumferentially around the steering unit. Different instruments can include different numbers of longitudinal elements depending on the deflection capabilities desired.

Any number of deflection or bending zones can be included by using an appropriate number of longitudinal elements and connecting them at appropriate positions along the length of instrument. The distal end 1305 and the longitudinal elements may be made by laser cutting the same longitudinal tube. The distal end may have any number of bendable zones

When the operation or procedure is completed, instrument 100 can be easily and quickly disconnected from steering device as it has been explained in relation to several embodiments.

Instrument 100 can then be discarded, with steering device available to use with a new, clean instrument 100 for further operations. In some embodiments, instrument 100 could be sent for cleaning and sanitization for preparing for reuse. In such embodiments, the ability to disconnect the part needing cleaning can help to ensure proper cleaning and sterilization can be done. For example, a disconnected instrument could be placed in a sanitization chamber, whereas an instrument which didn't disconnect may not be due to size and/or other sensitive parts.

As discussed in the background, past instruments that were reused needed to go through extensive cleaning and decontamination operations for safe reuse. This was a time consuming process that did not always eliminate all contaminants. Some steerable instruments were disposable to avoid the time, costs and risks of attempting cleaning, but disposing of a full instrument after every operation is quite costly. By using a steering device which can couple to and decouple from an instrument 100 in a quick, easy and secure manner, only the part of the instrument which experiences the contamination can be disposed of (or cleaned and sterilized in some situations), and other parts can be safely reused without the need for extensive decontamination processes. As steering portions of such instruments can be complicated and a relatively expensive part, being able to decouple the exposed portion of the instrument from the steering portion, and only disposing of the exposed portion is a great economic benefit and results in less waste. Using a quick and simple coupling between instrument and steering device allows for quick coupling and decoupling of a disposable instrument 100 to a reusable steering device at the location of use, and by non-trained or non-technical persons.

WO 2009/112060 A1, WO 2009/127236 A1, WO 2017/213491 A1, and WO 2018/067004 show embodiments in which this invention can be applied and are hereby incorporated by reference.

The instrument 100 may be manufactured such that the longitudinal elements 1302 are implemented by longitudinal strip shaped elements in a tube separated by longitudinal slots resulting from laser cutting in the cylindrical tube, and the bendable parts of the distal end may be implemented performing laser cuts in the same tube as explained in detail in, for example, WO 2009/112060 A1, WO 2009/127236 A1, WO 2017/213491 A1, and WO 2018/067004. As an alternative to laser cutting other techniques may be used, e.g., cutting by means of water jets. Also, other methods such as 3D laser printing may be used. These slots extend through the whole thickness of the material. This also holds for other embodiments discussed above.

The examples and embodiments described herein serve to illustrate rather than to limit the invention. Elements from different embodiments can be combined to form embodiments not shown in the Figures unless such combinations are non-compatible. The person skilled in the art will be able to design alternative embodiments without departing from the scope of the claims. Reference signs placed in parentheses in the claims shall not be interpreted to limit the scope of the claims. Items described as separate entities in the claims or the description may be implemented as a single item or multiple hardware items combining the features of the items described.

It is to be understood that the invention is limited by the annexed claims. In this document and in its claims, the verb "to comprise" and its conjugations are used in their non-limiting sense to mean that items following the word are included, without excluding items not specifically mentioned. In addition, reference to an element by the indefinite article "a" or "an" does not exclude the possibility that more than one of the element is present, unless the context clearly requires that there be one and only one of the elements. The indefinite article "a" or "an" thus usually means "at least one".

## Claims

1. A steering device (500, 700, 1200) for connecting to an elongated instrument comprising:
• a supporting unit (502) arranged around a first channel (508) having a first central axis, said first channel (508) extending from a distal side to a proximal side of the supporting unit; and
• a steering unit (504) rotationally arranged on said supporting unit (502), the steering unit (502) arranged to secure/release a plurality of longitudinal elements (102; 1102; 1302) made by cutting a plurality of slots (104) in a tube of an instrument and being provided with a connecting part (108; 1104 in the form of a wider portion (108; 1104) at their end, the steering unit comprising a plurality of grooves (532) extending inward from the circumference of the steering unit (504) or a plurality of openings (702) each groove or opening configured to receive one longitudinal element (102) and to secure/release the wider portion (108; 1104) of one of the plurality of longitudinal elements (102; 1102; 1302) such that the longitudinal elements can move with a rotational movement of the steering unit (504).

2. The steering device (500; 700; 1101; 1308) according to claim 1, wherein the supporting unit (502) comprises a ball shaped element (506) such that the steering unit (504) can rotate around the ball shaped element (506) and the first channel (508) extends through the ball shaped element (506).

3. The steering device (500; 700; 1101; 1308) according to any of the preceding claims, wherein the steering unit (504) comprises a steering plate (516).

4. The steering device (500; 700; 1308) according to claim 3, wherein the steering plate (516) comprises the plurality of grooves (532), the steering device further comprising a locking mechanism (804; 1002; 1103) for securing the longitudinal elements (102; 1102; 1302) to the steering unit (504).

5. The steering device (500; 700; 1302) of claim 4, wherein the locking mechanism comprises a locking plate (804; 1002) configured to secure the plurality of longitudinal elements (102; 1102; 1302) to the steering unit (504).

6. The steering device (500, 700; 1302) of claim 5, wherein the plurality of longitudinal elements (102; 1102; 1302) can be secured to the steering unit (504) through the plurality of grooves (532) and the locking plate (804; 1002) connects to the steering unit such that the locking plate can prevent the plurality of longitudinal elements from exiting the plurality of grooves.

7. The steering device of claim 6, wherein the locking plate (804; 1002) is movable from an open position where the longitudinal elements can be inserted or removed from the grooves (532) to a locked position where the longitudinal elements are secured within the grooves (532).

8. The steering device of claim 7, wherein the locking plate (804) is movable axially to secure longitudinal elements in the grooves (532) extending inward from the circumference of the steering unit.

9. The steering device of claim 7, wherein the locking plate (1002) is rotationally moveable to move from the open position to the locked position.

10. The steering device of claim 9, wherein the locking plate (1002) comprises a plurality of protruding elements (1004) extending around the outer circumference of the locking plate, wherein each of the plurality of protruding elements comprises a hook-shaped side arranged to receive a longitudinal element in a channel to secure the longitudinal element.

11. The steering device of claim 10, wherein each of the plurality of protruding elements (1004) comprises an inclined side arranged opposite the channel such that when the locking plate rotates from the locked position with the longitudinal elements secured in the channels to the open position, the longitudinal elements slide on the inclined side of the protruding elements and are thereby pushed out from the plurality of openings.

12. The steering device of any of the preceding claims, wherein the steering device is arranged such that, when the plurality of longitudinal elements are secured to the steering unit, rotational movement of the steering unit around the supporting unit will cause the steering unit to push and/or pull the plurality of longitudinal elements.

13. The steering device of any of the preceding claims, and further comprising a guiding element for guiding the plurality of elongated elements into securing positions of the steering unit.

14. The steering device according to claim 3, wherein the steering plate (516) comprises the said plurality of openings (702), each opening being provided with a ramp to guide the wider portion of one of the plurality of longitudinal elements to a securing position.

15. An elongated instrument for connecting to a steering device, the instrument comprising:
a tube with a distal end and a proximal end; and
a plurality of elongated elements (102; 1102; 1302) extending along the tube and made by cutting a plurality of slots (104) in the tube, each of the plurality of elongated elements comprising at the proximal end a connecting part (108; 1104; 1204) for securing the elongated instrument to the steering device, the connecting part having the form of a wider portion (108; 1104) at the end of the longitudinal element configured to be received in one of a plurality of grooves (532) extending inward from the circumference of the steering unit (504) or in a plurality of openings (702) each groove or opening configured to secure/release the wider portion (108; 1104) of one of the plurality of longitudinal elements (102; 1102; 1302) such that the longitudinal elements can move with a rotational movement of the steering unit (504).

16. The elongated instrument of claim 15, wherein the plurality of longitudinal elements are arranged extending outwardly from a central axis (103; 806) of the tube at the proximal end of the tube.

17. The elongated instrument of claim 16 comprising a cone-shaped device (402; 1106) at the proximal end arranged to push away the longitudinal elements for extending outwardly from the elongated shaft.

18. The elongated instrument of any of the claims 15-17, wherein the slots in the tube are made by either laser or water cutting.

19. The elongated instrument of any of the claims 15-18, wherein the instrument comprises at least a distal deflectable zone at a distal end of the instrument, wherein the longitudinal elements are connected to the distal deflectable zone such that by pulling or pushing the longitudinal elements the distal deflectable zone is controlled to deflect in a desired direction.

20. A medical device comprising the elongated instrument according to any of claims 15-19 and the steering device according to any of claims 1-14.

## Patentansprüche

1. Lenkvorrichtung (500, 700, 1200) zum Verbinden mit einem länglichen Instrument, umfassend:
• eine Stützeinheit (502), die um einen ersten Kanal (508), der eine erste Mittelachse aufweist, angeordnet ist, wobei sich der erste Kanal (508) von einer distalen Seite zu einer proximalen Seite der Stützeinheit erstreckt; und
• eine Lenkeinheit (504), die auf der Stützeinheit (502) drehbar angeordnet ist, wobei die Lenkeinheit (502) angeordnet ist, um eine Vielzahl von Längselementen (102; 1102; 1302), die durch Schneiden einer Vielzahl von Schlitzen (104) in ein Rohr eines Instruments hergestellt sind und an ihrem Ende mit einem Verbindungsteil (108; 1104 in der Form eines breiteren Abschnitts (108; 1104) versehen sind, zu sichern/freizugeben, die Lenkeinheit umfassend eine Vielzahl von Nuten (532), die sich von dem Umfang der Lenkeinheit (504) nach innen erstrecken, oder eine Vielzahl von Öffnungen (702), wobei jede Nut oder Öffnung konfiguriert ist, um ein Längselement (102) aufzunehmen und den breiteren Abschnitt (108; 1104) eines der Vielzahl von Längselementen (102; 1102; 1302) derart zu sichern/freizugeben, dass sich die Längselemente mit einer Drehbewegung der Lenkeinheit (504) bewegen können.

2. Lenkvorrichtung (500; 700; 1101; 1308) nach Anspruch 1, wobei die Stützeinheit (502) ein kugelförmiges Element (506) derart umfasst, dass sich die Lenkeinheit (504) um das kugelförmige Element (506) drehen kann und sich der erste Kanal (508) durch das kugelförmige Element (506) erstreckt.

3. Lenkvorrichtung (500; 700; 1101; 1308) nach einem der vorstehenden Ansprüche, wobei die Lenkeinheit (504) eine Lenkplatte (516) umfasst.

4. Lenkvorrichtung (500; 700; 1308) nach Anspruch 3, wobei die Lenkplatte (516) die Vielzahl von Nuten (532) umfasst, die Lenkvorrichtung ferner umfassend ein Gesperre (804; 1002; 1103) zum Sichern der Längselemente (102; 1102; 1302) an der Lenkeinheit (504).

5. Lenkvorrichtung (500; 700; 1302) nach Anspruch 4, wobei das Gesperre eine Gesperreplatte (804; 1002), die konfiguriert ist, um die Vielzahl von Längselementen (102; 1102; 1302) an der Lenkeinheit (504) zu sichern, umfasst.

6. Lenkvorrichtung (500, 700; 1302) nach Anspruch 5, wobei die Vielzahl von Längselementen (102; 1102; 1302) durch die Vielzahl von Nuten (532) an der Lenkeinheit (504) gesichert werden können und die Gesperreplatte (804; 1002) mit der Lenkeinheit derart verbunden ist, dass die Gesperreplatte verhindern kann, dass die Vielzahl von Längselementen aus der Vielzahl von Nuten austreten.

7. Lenkvorrichtung nach Anspruch 6, wobei die Gesperreplatte (804; 1002) von einer offenen Position, in der die Längselemente in die Nuten (532) eingeführt oder aus diesen entfernt werden können, in eine gesperrte Position, in der die Längselemente innerhalb der Nuten (532) gesichert sind, bewegbar ist.

8. Lenkvorrichtung nach Anspruch 7, wobei die Gesperreplatte (804) axial bewegbar ist, um Längselemente in den Nuten (532), die sich von dem Umfang der Lenkeinheit nach innen erstrecken, zu sichern.

9. Lenkvorrichtung nach Anspruch 7, wobei die Gesperreplatte (1002) drehbar bewegbar ist, um sich von der offenen Position in die gesperrte Position zu bewegen.

10. Lenkvorrichtung nach Anspruch 9, wobei die Gesperreplatte (1002) eine Vielzahl von vorstehenden Elementen (1004), die sich um den Außenumfang der Gesperreplatte erstrecken, umfasst, wobei jedes der Vielzahl von vorstehenden Elementen eine hakenförmige Seite, die angeordnet ist, um ein Längselement in einem Kanal aufzunehmen, um das Längselement zu sichern, umfasst.

11. Lenkvorrichtung nach Anspruch 10, wobei jedes der Vielzahl von vorstehenden Elementen (1004) eine geneigte Seite, die gegenüber dem Kanal derart angeordnet ist, dass, wenn sich die Gesperreplatte von der gesperrten Position mit den in den Kanälen gesicherten Längselementen in die offene Position dreht, die Längselemente auf der geneigten Seite der vorstehenden Elemente gleiten und dadurch aus der Vielzahl von Öffnungen herausgeschoben werden, umfasst.

12. Lenkvorrichtung nach einem der vorstehenden Ansprüche, wobei die Lenkvorrichtung derart angeordnet ist, dass, wenn die Vielzahl von Längselementen an der Lenkeinheit gesichert sind, die Drehbewegung der Lenkeinheit um die Stützeinheit die Lenkeinheit veranlassen wird, die Vielzahl von Längselementen zu schieben und/oder zu ziehen.

13. Lenkvorrichtung nach einem der vorstehenden Ansprüche, und ferner umfassend ein Führungselement zum Führen der Vielzahl von länglichen Elementen in Sicherungspositionen der Lenkeinheit.

14. Lenkvorrichtung nach Anspruch 3, wobei die Lenkplatte (516) die Vielzahl von Öffnungen (702) umfasst, wobei jede Öffnung mit einer Rampe versehen ist, um den breiteren Abschnitt eines der Vielzahl von Längselementen in eine Sicherungsposition zu führen.

15. Längliches Instrument zum Verbinden mit einer Lenkvorrichtung, das Instrument umfassend:
ein Rohr mit einem distalen Ende und einem proximalen Ende; und
eine Vielzahl von länglichen Elementen (102; 1102; 1302), die sich entlang des Rohrs erstrecken und durch Schneiden einer Vielzahl von Schlitzen (104) in das Rohr hergestellt sind, jedes der Vielzahl von länglichen Elementen umfassend an dem proximalen Ende ein Verbindungsteil (108; 1104; 1204) zum Sichern des länglichen Instruments an der Lenkvorrichtung, wobei das Verbindungsteil die Form eines breiteren Abschnitts (108; 1104) an dem Ende des Längselements aufweist, das konfiguriert ist, um in einer von einer Vielzahl von Nuten (532), die sich von dem Umfang der Lenkeinheit (504) nach innen erstrecken, oder in einer Vielzahl von Öffnungen (702) aufgenommen zu werden, wobei jede Nut oder Öffnung konfiguriert ist, um den breiteren Abschnitt (108; 1104) eines der Vielzahl von Längselementen (102; 1102; 1302) derart zu sichern/freizugeben, dass sich die Längselemente mit einer Drehbewegung der Lenkeinheit (504) bewegen können.

16. Längliches Instrument nach Anspruch 15, wobei die Vielzahl von Längselementen sich von einer Mittelachse (103; 806) des Rohrs an dem proximalen Ende des Rohrs nach außen erstreckend angeordnet sind.

17. Längliches Instrument nach Anspruch 16, umfassend eine kegelförmige Vorrichtung (402; 1106) an dem proximalen Ende, die angeordnet ist, um die Längselemente zum Erstrecken nach außen von dem länglichen Schaft wegzudrücken.

18. Längliches Instrument nach einem der Ansprüche 15 bis 17, wobei die Schlitze in dem Rohr entweder durch Laser- oder Wasserschneiden hergestellt sind.

19. Längliches Instrument nach einem der Ansprüche 15 bis 18, wobei das Instrument mindestens eine distale auslenkbare Zone an einem distalen Ende des Instruments umfasst, wobei die Längselemente mit der distalen auslenkbaren Zone derart verbunden sind, dass durch Ziehen oder Drücken der Längselemente die distale auslenkbare Zone gesteuert wird, um in eine gewünschte Richtung ausgelenkt zu werden.

20. Medizinische Vorrichtung, umfassend das längliche Instrument nach einem der Ansprüche 15 bis 19 und die Lenkvorrichtung nach einem der Ansprüche 1 bis 14.

## Revendications

1. Dispositif de direction (500, 700, 1200) destiné à être connecté à un instrument allongé comprenant :
• une unité de support (502) agencée autour d'un premier canal (508) ayant un premier axe central, ledit premier canal (508) s'étendant d'un côté distal à un côté proximal de l'unité de support ; et
• une unité de direction (504) agencée de manière rotative sur ladite unité de support (502), l'unité de direction (502) étant agencée pour fixer/libérer une pluralité d'éléments longitudinaux (102 ; 1102 ; 1302) fabriqués en découpant une pluralité de fentes (104) dans un tube d'un instrument et étant pourvus d'une partie de connexion (108 ; 1104 sous la forme d'une partie plus large (108 ; 1104) à leur extrémité, l'unité de direction comprenant une pluralité de rainures (532) s'étendant vers l'intérieur depuis la circonférence de l'unité de direction (504) ou une pluralité d'ouvertures (702) chaque rainure ou ouverture étant configurée pour recevoir un élément longitudinal (102) et pour fixer/libérer la partie plus large (108 ; 1104) de l'un de la pluralité d'éléments longitudinaux (102 ; 1102 ; 1302) de telle sorte que les éléments longitudinaux peuvent se déplacer avec un mouvement de rotation de l'unité de direction (504).

2. Dispositif de direction (500 ; 700 ; 1101 ; 1308) selon la revendication 1, dans lequel l'unité de support (502) comprend un élément en forme de bille (506) de telle sorte que l'unité de direction (504) peut tourner autour de l'élément en forme de bille (506) et que le premier canal (508) s'étend à travers l'élément en forme de bille (506).

3. Dispositif de direction (500 ; 700 ; 1101 ; 1308) selon l'une quelconque des revendications précédentes, dans lequel l'unité de direction (504) comprend une plaque de direction (516).

4. Dispositif de direction (500 ; 700 ; 1308) selon la revendication 3, dans lequel la plaque de direction (516) comprend la pluralité de rainures (532), le dispositif de direction comprenant en outre un mécanisme de verrouillage (804 ; 1002 ; 1103) pour fixer les éléments longitudinaux (102 ; 1102 ; 1302) à l'unité de direction (504).

5. Dispositif de direction (500 ; 700 ; 1302) selon la revendication 4, dans lequel le mécanisme de verrouillage comprend une plaque de verrouillage (804 ; 1002) configurée pour fixer la pluralité d'éléments longitudinaux (102 ; 1102 ; 1302) à l'unité de direction (504).

6. Dispositif de direction (500, 700 ; 1302) selon la revendication 5, dans lequel la pluralité d'éléments longitudinaux (102 ; 1102 ; 1302) peuvent être fixés à l'unité de direction (504) à travers la pluralité de rainures (532) et la plaque de verrouillage (804 ; 1002) se connecte à l'unité de direction de telle sorte que la plaque de verrouillage peut empêcher la pluralité d'éléments longitudinaux de sortir de la pluralité de rainures.

7. Dispositif de direction selon la revendication 6, dans lequel la plaque de verrouillage (804 ; 1002) est mobile d'une position ouverte dans laquelle les éléments longitudinaux peuvent être insérés ou retirés des rainures (532) vers une position verrouillée dans laquelle les éléments longitudinaux sont fixés à l'intérieur des rainures (532).

8. Dispositif de direction selon la revendication 7, dans lequel la plaque de verrouillage (804) est mobile axialement pour fixer des éléments longitudinaux dans les rainures (532) s'étendant vers l'intérieur depuis la circonférence de l'unité de direction.

9. Dispositif de direction selon la revendication 7, dans lequel la plaque de verrouillage (1002) est mobile en rotation pour se déplacer de la position ouverte à la position verrouillée.

10. Dispositif de direction selon la revendication 9, dans lequel la plaque de verrouillage (1002) comprend une pluralité d'éléments en saillie (1004) s'étendant autour de la circonférence extérieure de la plaque de verrouillage, dans lequel chacun de la pluralité d'éléments en saillie comprend un côté en forme de crochet agencé pour recevoir un élément longitudinal dans un canal pour fixer l'élément longitudinal.

11. Dispositif de direction selon la revendication 10, dans lequel chacun de la pluralité d'éléments en saillie (1004) comprend un côté incliné agencé à l'opposé du canal de telle sorte que lorsque la plaque de verrouillage tourne de la position verrouillée avec les éléments longitudinaux fixés dans les canaux vers la position ouverte, les éléments longitudinaux glissent sur le côté incliné des éléments en saillie et sont ainsi poussés hors de la pluralité d'ouvertures.

12. Dispositif de direction selon l'une quelconque des revendications précédentes, dans lequel le dispositif de direction est agencé de telle sorte que, lorsque la pluralité d'éléments longitudinaux est fixée à l'unité de direction, un mouvement de rotation de l'unité de direction autour de l'unité de support amène l'unité de direction à pousser et/ou tirer la pluralité d'éléments longitudinaux.

13. Dispositif de direction selon l'une quelconque des revendications précédentes, et comprenant en outre un élément de guidage pour guider la pluralité d'éléments allongés dans des positions de fixation de l'unité de direction.

14. Dispositif de direction selon la revendication 3, dans lequel la plaque de direction (516) comprend ladite pluralité d'ouvertures (702), chaque ouverture étant pourvue d'une rampe pour guider la partie plus large de l'un de la pluralité d'éléments longitudinaux vers une position de fixation.

15. Instrument allongé destiné à être connecté à un dispositif de direction, l'instrument comprenant :
un tube ayant une extrémité distale et une extrémité proximale ; et
une pluralité d'éléments allongés (102 ; 1102 ; 1302) s'étendant le long du tube et réalisés par la découpe d'une pluralité de fentes (104) dans le tube, chacun de la pluralité d'éléments allongés comprenant à l'extrémité proximale une partie de connexion (108 ; 1104 ; 1204) pour fixer l'instrument allongé au dispositif de direction, la partie de connexion présentant
la forme d'une partie plus large (108 ; 1104) à l'extrémité de l'élément longitudinal configurée pour être reçue dans l'une d'une pluralité de rainures (532) s'étendant vers l'intérieur depuis la circonférence de l'unité de direction (504) ou dans une pluralité d'ouvertures (702) chaque rainure ou ouverture étant configurée pour fixer/libérer la partie plus large (108 ; 1104) de l'un de la pluralité d'éléments longitudinaux (102 ; 1102 ; 1302) de telle sorte que les éléments longitudinaux peuvent se déplacer avec un mouvement de rotation de l'unité de direction (504).

16. Instrument allongé selon la revendication 15, dans lequel la pluralité d'éléments longitudinaux est agencée de manière à s'étendre vers l'extérieur depuis un axe central (103 ; 806) du tube à l'extrémité proximale du tube.

17. Instrument allongé selon la revendication 16 comprenant un dispositif en forme de cône (402 ; 1106) à l'extrémité proximale agencé pour repousser les éléments longitudinaux pour s'étendre vers l'extérieur à partir de la tige allongée.

18. Instrument allongé selon l'une quelconque des revendications 15 à 17, dans lequel les fentes dans le tube sont réalisées par découpe au laser ou à l'eau.

19. Instrument allongé selon l'une quelconque des revendications 15 à 18, dans lequel l'instrument comprend au moins une zone défléchissable distale à une extrémité distale de l'instrument, dans lequel les éléments longitudinaux sont reliés à la zone défléchissable distale de telle sorte qu'en tirant ou en poussant les éléments longitudinaux, la zone défléchissable distale est commandée pour se défléchir dans une direction souhaitée.

20. Dispositif médical comprenant l'instrument allongé selon l'une quelconque des revendications 15 à 19 et le dispositif de direction selon l'une quelconque des revendications 1 à 14.
